Europäisches Patentamt

European Patent Office

Office européen des brevets

(19)

(11) Veröffentlichungsnummer : **0 116 849**
**B1**

(12) **EUROPÄISCHE PATENTSCHRIFT**

(45) Veröffentlichungstag der Patentschrift :
**30.07.86**

(21) Anmeldenummer : **84100386.6**

(22) Anmeldetag : **16.01.84**

(51) Int. Cl.⁴ : **A 61 F 13/20**

(54) Verfahren zum Herstellen eines Wattetampons.

(30) Priorität : **24.01.83 DE 3302193**

(43) Veröffentlichungstag der Anmeldung :
**29.08.84 Patentblatt 84/35**

(45) Bekanntmachung des Hinweises auf die Patenterteilung : **30.07.86 Patentblatt 86/31**

(84) Benannte Vertragsstaaten :
**AT BE CH DE FR GB IT LI NL SE**

(56) Entgegenhaltungen :
**DE-A- 2 722 802**
**DE-B- 1 945 427**
**DE-B- 2 445 214**
**DE-C- 1 076 885**
**FR-A- 2 232 297**
**US-A- 2 188 923**
**US-A- 2 926 667**

(73) Patentinhaber : **Vereinigte Papierwerke Schickedanz & Co.**
**Schoppershofstrasse 50**
**D-8500 Nürnberg (DE)**

(72) Erfinder : **Siegers, Hans Peter**
**Schwalmweg 18**
**D-5144 Wegberg (DE)**

**Beschreibung**

Die Erfindung betrifft ein Verfahren zum Herstellen eines Wattetampons mit Rückholkordel, bei dem ein Wattebandabschnitt mit einem flusenfreien Hüllmaterial, insbesondere aus Vliesstoff, umschlossen wird.

Aus DE-A-27 22 802 ist ein gattungsgemäßes Verfahren bekannt, wobei eine Menge von durch Faserabfälle gebildetem absorbierendem Stoff in eine dehnbare Hülle eingeschlossen wird, welche aus dehnbarer Gaze besteht und wobei dem ganzen eine zylindrische Form gegeben wird. Bei den auf diese Weise herstellbaren Tampons ist es nachteilig, daß die Möglichkeit der Flusenbildung nicht ausreichend sicher verhindert werden kann. Es können deshalb insbesondere bei in der Frauenhygiene verwendeten Tampons Fasern bzw. Wattebestandteile in der Vagina zurückbleiben.

Der Erfindung liegt demgegenüber die Aufgabe zugrunde, den Wattebandabschnitt so mit dem Hüllmaterial — zwecks Vermeidens eines Abflusens — zu umschließen, daß in dem die Rückholkordel aufweisenden hinteren Ende gegenüberliegenden Kopfbereich des Tampons sich weder Materialanhäufungen — wie beim Verpressen — noch Siegelnähte — wie beim Rundumsiegeln — befinden.

Die erfindungsgemäße Lösung besteht bei dem eingangs genannten Verfahren in der Kombination der Maßnahmen gemäß Hauptanspruch. Zweckmäßige Ausgestaltungen sind Gegenstand der Unteransprüche.

Ein erfindungsgemäß hergestellter Tampon hat im Kopfbereich eine für die Flüssigkeitsaufnahme sehr günstige Form, da sich dort keine Materialanhäufungen und keine Siegelnähte befinden. Ein weiterer Vorteil des erfindungsgemäßen Verfahrens besteht darin, daß die zum Einbringen des Hüllmaterialvorhangs erforderliche Vorrichtung, insbesondere die Abrollmittel und Messer, platzsparend in die Tamponmaschine zu integrieren ist.

Der erfindungsgemäß mit Hüllmaterial einzuschlagende Wattebandabschnitt besitzt ähnlich wie bei der Vorbereitung zum Herstellen von Preßtampons zwei oder vier Schnittkanten. Als Hüllmaterial kann vorzugsweise siegelfähiger Vliesstoff in der Gewichtsklasse von 10 bis 20 g/m². eingesetzt werden.

Der jeweilige von einem Watteband abzutrennende Abschnitt kann unmittelbar vor dem Aufnähen der Rückholkordel in das Hüllmaterial eingeschoben werden. Es ist jedoch günstiger, die Rückholkordel auf den Wattebandabschnitt aufzubringen, insbesondere aufzunähen, bevor dieser in das Hüllmaterial·eingeschoben wird. Im letzteren Fall liegt nämlich die Rückholkordel innerhalb des Hüllvlieses und erscheint daher nicht auf der Außenfläche und insbesondere nicht am Kopf des fertigen Tampons.

Anhand der schematischen Darstellung eines Ausführungsbeispiels werden Einzelheiten der Erfindung erläutert. Es zeigen :

Figur 1  Verfahrensschritte beim Herstellen eines Tampons mit außenliegender Rückholkordel ; und

Figur 2  Verfahrensschritte beim Herstellen eines Tampons mit innenliegender Rückholkordel.

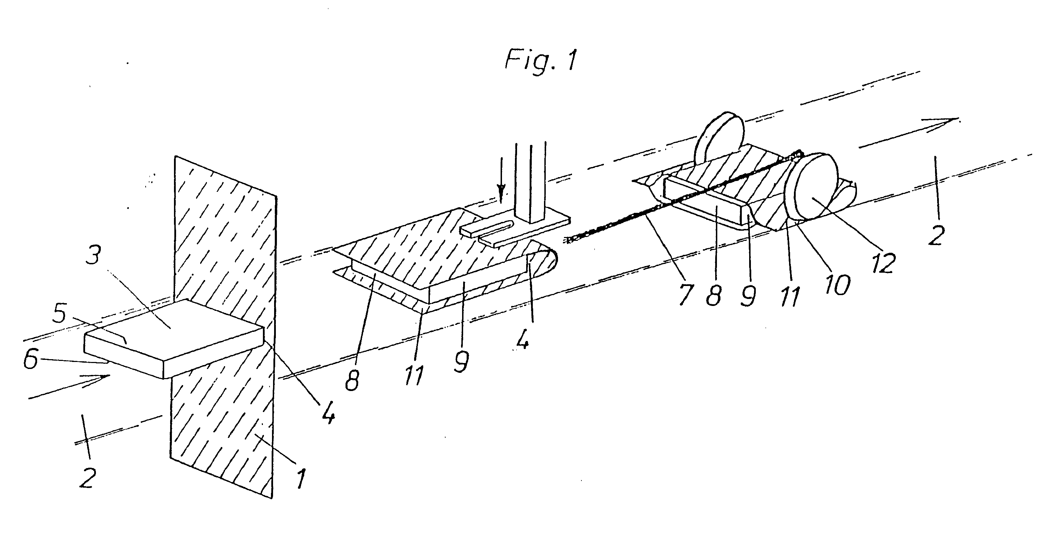

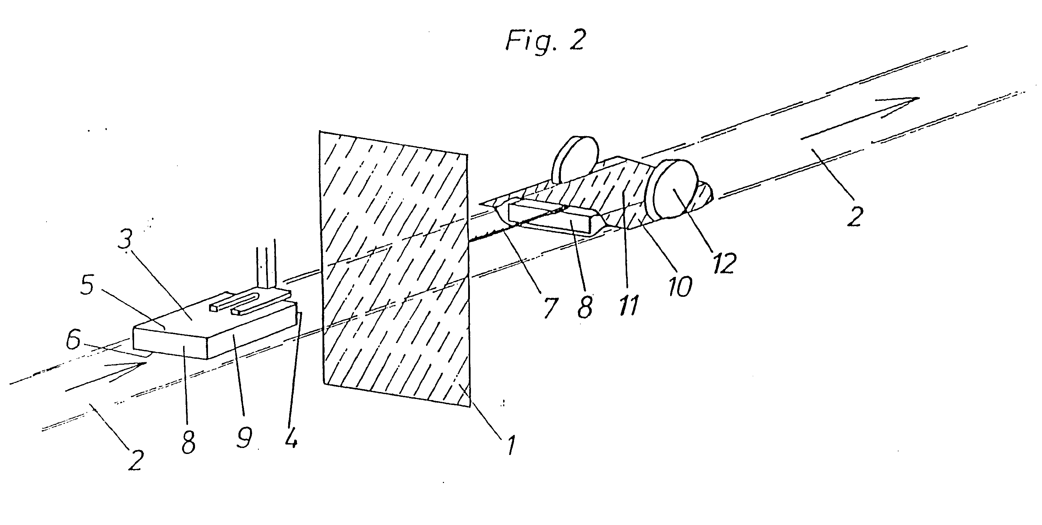

Bei den in Fig. 1 und 2 angedeuteten Verfahrensschritten wird das Hüllmaterial 1 nach Art eines Vorhangs vertikal in den durch einen Pfeil angedeuteten Transportweg 2 des bereits vorher von einem Watteband abgetrennten Wattebandabschnitts 3 gespannt. Letzterer wird mit seiner Kopfkante 4 mittig in den Hüllmaterialvorhang 1 geschoben, derart, daß sich das Hüllmaterial über Oberseite 5 und Unterseite 6 des Wattebandabschnitts legt. Bei der Verfahrensalternative nach Fig. 1 erfolgt das Einschieben des Wattebandabschnitts 3 in den Hüllmaterialvorhang 1 vor dem Aufnähen der Rückholkordel 7. Die Rückholkordel 7 liegt dann auf der Außenfläche des fertigen Produkts. Bei der Verfahrensalternative nach Fig. 2 wird das Aufnähen der Rückholkordel 7 vorgenommen, bevor der mit Rückholkordel 7 versehene Wattebandabschnitt in den Hüllmaterialvorhang 1 eingeschoben wird. In diesem Fall tritt die Rückholkordel 7 am fertigen Produkt nur am hinteren Ende 8 zu Tage.

Die an den Seitenkanten 9 überstehenden Seitenränder 10 des auf den Wattebandabschnitt 3 gefalteten Hüllmaterials 11 werden in den dargestellten Ausführungsbeispielen mit Hilfe von Siegelrollen 12 verschlossen. Anstelle der Siegelrollen 12 können auch Siegelzangen, Leim oder andere Mittel zum stoffschlüssigen Verbinden eingesetzt werden. Sollte es erforderlich sein, das hintere Ende 8 des Wattebandabschnitts 3 zu schließen, wird das Hüllmaterialstück so verlängert vorgelegt, daß es auf der Ober- und Unterseite des Wattebandabschnitts 3 um größenordnungsmäßig 5 mm übersteht. Sollte ein Verpressen dieses überstehenden Materials gegen das Tamponende nicht ausreichen, kann durch einen Siegelbalken, eine Verleimung oder dergleichen auch in diesem Bereich eine stoffschlüssige Verbindung vorgesehen werden.

**Patentansprüche**

1. Verfahren zum Herstellen eines Wattetampons mit Rückholkordel (7), bei dem ein Wattebandabschnitt (3) mit einem flusenfreien Hüllmaterial (11), insbesondere aus Vliesstoff, umschlossen wird, dadurch gekennzeichnet, daß das Watteband vor dem Einschieben in das Hüllmaterial (11) in Abschnitte (3) endgültiger Größe abgeteilt wird, daß das Hüllmaterial (11) nach Art eines Vorhangs (1) vertikal in den Transportweg (2) des Wattebandabschnittes (3) mittig vor dessen Kopfkante (4) gespannt wird, daß der Hüllmaterialvorhang (1) durch Verschieben des Wattebandabschnittes (3) um dessen Kopfkante (4) gefaltet sowie auf dessen obere und untere Fläche (5, 6) gelegt wird und daß die an den sich seitlich an die Kopfkante (4) anschliessenden Seitenkanten (9) des Wattebandabschnittes (3) aneinanderstoßenden Ränder (10) des Hüllmaterials (11) stoffschlüssig flusendicht miteinander verbunden werden.

2. Verfahren nach Anspruch 1, dadurch gekennzeichnet, daß die Rückholkordel (7) von dem Einschieben in das Hüllmaterial (11) auf den Wattebandabschnitt (3) aufgebracht, insbesondere aufgenäht, wird.

3. Verfahren nach Anspruch 1 oder 2, dadurch gekennzeichnet, daß das Hüllmaterial (11) im Hinblick auf ein Überstehen um größenordnungsmäßig 5 mm an dem der Kopfkante (4) gegenüberliegenden hinteren Ende (8) des Wattebandabschnitts (3) verlängert wird.

4. Verfahren nach Anspruch 3, dadurch gekennzeichnet, daß die an dem hinteren Ende (8) überstehenden Hüllmaterialränder gegen das Tamponende verpreßt werden.

5. Verfahren nach Anspruch 3, dadurch gekennzeichnet, daß die an dem hinteren Ende (8) überstehenden Hüllmaterialränder stoffschlüssig verbunden werden.

## Claims

1. Method for the production of a cotton wool tampon with retraction chord (7), in which a cotton wool tape portion (3) is enclosed by a fluff-free covering material (11), in particular of fleece fabric, characterised thereby, that the cotton wool tape is divided into portions (3) of final size before being pushed into the covering material (11), that the covering material (11) is drawn in the manner of a curtain (1) vertically into the transport path (2) of the cotton wool tape portion (3) centrally in front of its leading edge (4), that the covering material curtain (1) is folded through displacement of the cotton wool tape portion (3) around its leading edge (4) as well as laid onto its upper and lower surface (5, 6) and that the margins (10) of the covering material (11), which each abut against the other at those side edges (9) of the cotton wool tape portion (3), which adjoin laterally at the leading edge (4), are each connected with the other in substance-locking and fluff-tight manner.

2. Method according to claim 1, characterised thereby, that the retraction chord (7) is mounted, in particular sewn onto the cotton wool tape portion (3) before being pushed into the covering material (11).

3. Method according to claim 1 or 2, characterised thereby, that the covering material (11) is prolonged at the rear end (8), lying opposite the leading edge (4), of the cotton wool tape portion (3), by the order of magnitude of 5 millimetres with a view to a protruding.

4. Method according to claim 3, characterised thereby, that the covering material margins protruding at the rear end (8) are pressed together against the end of the tampon.

5. Method according to claim 3, characterised thereby, that the covering material margins protruding at the rear end (8) are connected in substance-locking manner.

## Revendications

1. Procédé pour la fabrication d'un tampon d'ouate avec cordon de rappel (7), dans lequel on enferme un tronçon de bande d'ouate (3) avec une matière d'enveloppe exempte de duvets (11), en particulier constituée par du tissu de laine, caractérisé par le fait qu'avant de l'enfoncer dans la matière d'enveloppe, on divise la bande d'ouate en tronçons (3) de grandeur définitive ; que l'on tend verticalement la matière d'enveloppe (11) à la manière d'un rideau (1) dans le parcours de transport (2) du tronçon de bande d'ouate (3), le bord de tête (4) de ce dernier étant situé au niveau du milieu du rideau (1) ; que l'on plie ce rideau (1) de matière d'enveloppe (11), par glissement du tronçon de bande d'ouate (3), avec son bord de tête (4) s'engageant dans ce rideau de manière que ses moitiés supérieure et inférieure se plaquent sur les surfaces supérieure et inférieure (5, 6) correspondantes du tronçon ; et que l'on relie entre eux, de façon solidaire quant à la matière et étanche quant aux duvets, les bords (10) de la matière d'enveloppe (11), lesquels se raccordent aux bords latéraux (9) du tronçon de bande d'ouate (3) qui se rattachent latéralement au bord de tête (4).

2. Procédé selon la revendication 1, caractérisé par le fait qu'avant d'engager le tronçon de bande d'ouate (3) dans la matière d'enveloppe (11), on fixe le cordon de rappel (7) sur ce tronçon, en particulier par couture.

3. Procédé selon l'une ou l'autre des revendications 1 ou 2, caractérisé par le fait que l'on allonge la matière d'enveloppe (11), en vue d'un dépassement d'un ordre de grandeur de 5 mm, à l'extrémité postérieure (8) du tronçon de bande d'ouate (3), c'est-à-dire à l'extrémité opposée au bord de tête (4) de ce dernier.

4. Procédé selon la revendication 3, caractérisé par le fait que l'on comprime contre l'extrémité postérieure (8) du tampon les bords correspondants de la matière d'enveloppe qui dépassent de cette extrémité.

5. Procédé selon la revendication 3, caractérisé par le fait que l'on relie de façon solidaire quant à la matière les bords de la matière d'enveloppe qui dépassent de l'extrémité postérieure (8) du tampon.

Fig. 1

Fig. 2